# EUROPEAN PATENT APPLICATION

(11) **EP 4 180 517 A1**
(43) Date of publication of application: **17.05.2023**
(21) Application number: 22204961.1
(22) Date of filing: 01.11.2022
(51) Int. Cl.: C12N 5/077, C12N 5/0775

(54) **PRE-CONDITIONED MESENCHYMAL STEM CELLS AND PREPARATIONS AND APPLICATIONS THEREOF**

(30) Priority: 11.11.2021 US 202163278107 P
(71) Applicant: Buddhist Tzu Chi Medical Foundation, Hualien City, 970 (TW)
(72) Inventor: HUANG, CHIH-YANG, 970 Hualien City (TW); LIN, YU-JUNG, 970 Hualien City (TW); CHEN, SHAO-TSU, 970 Hualien City (TW); LIN, TZU-YING, 970 Hualien City (TW)
(74) Representative: Betten & Resch

(57) **Abstract**

Provided is a pre-conditioned mesenchymal stem cell (MSC), an exosome derived therefrom, and a cell-protective composition including the pre-conditioned MSC or the exosome. Also provided is a method for preparing the pre-conditioned MSC by contacting an MSC with an effective amount of ginkgolide A. Still provided is a method for promoting recovery or reducing death of damaged nerve cells, including administering to the damaged nerve cells a composition including the pre-conditioned MSC or the exosome.

## Description

### CROSS-REFERENCE TO RELATED APPLICATIONS

This application claims priority of U.S. Provisional Application No. 63/278,107, filed on November 11, 2021, the content of which is incorporated herein in its entirety by reference.

### BACKGROUND OF THE INVENTION

The present invention relates to stem cells and therapeutic uses thereof. Particularly, the present invention relates to a method for preparing a pre-conditioned mesenchymal stem cell (MSC) or an exosome derived therefrom, the pre-conditioned MSC or the exosome obtained thereby, and a method for promoting recovery or reducing death of damaged nerve cells by utilizing the pre-conditioned MSC or the exosome.

Parkinson's disease (PD) is one of the most common neurodegenerative disorders. It is estimated that more than 2% of the elderly population suffers from Parkinson's disease, which is expected to rise with the aging of the worldwide population. Some reports indicated that PD would become a pandemic. The pathological features of PD include the loss of midbrain dopamine (mDA) neurons or neuronal cell loss in the substantia nigra (SN), decreased dopamine secretion, and Lewy body accumulation in other brain tissues, thereby exacerbating motor impairment. One of the most effective treatments for PD is deep brain stimulation (DBS) through surgery. However, DBS attenuates PD progression marginally and does not improve neuronal cell death. Other treatments for PD include administering enzyme inhibitors and levodopa. Still, long-term drug usage may reduce therapeutic efficacy and lead to side effects involving involuntary motor action and dyskinesia, affecting patients' quality of life.

Studies on stem cell therapy in PD have been popular for decades. Mesenchymal stem cells are the most appropriate for stem cell therapy because of their differentiation ability and low immunogenicity. MSCs can be isolated from various tissues, including bone marrow, adipose tissue, umbilical cord Wharton's Jelly, and even the dental pulp, and thus have inspired researchers to find their abilities in cell transplantation therapy for PD. Increasing evidence has revealed that extracellular vesicles secreted by stem cells are essential in mediating tissue regeneration and may serve as a substitute for MSCs. Extracellular vesicles from MSCs include exosomes, which are small vesicles carrying genetic substances, proteins, enzymes, and growth factors. Exosomes from MSCs have been found to regulate different cell signaling pathways in target cells and promote their biological activities by transmitting information and content to improve disease conditions.

Though the MSC therapy is a promising tool in treating neurogenerative diseases such as PD, the therapeutic efficacy of MSCs largely depends on factors such as their ability to self-renew and migrate to injured tissues (homing) and their secretome. Accordingly, it is necessary to develop specific strategies to modulate the properties of MSCs so as to improve their therapeutic applications.

### SUMMARY OF THE INVENTION

The present invention concerns a pre-conditioned mesenchymal stem cell (MSC) or an exosome derived therefrom that could potentially treat neurodegenerative diseases. The pre-conditioned mesenchymal stem cell is an isolated mesenchymal stem cell pre-exposed to ginkgolide A (denoted as GA). GA is a compound purified from Ginkgo biloba and is represented by formula (I) below.

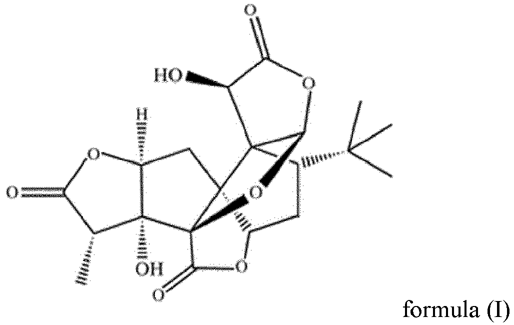

In some embodiments, the mesenchymal stem cell is derived from bone marrow, blood, adipose tissues, muscle, skin, dental pulp, umbilical cord tissues, placenta, amniotic fluid in a subject. In some embodiments, the mesenchymal stem cell is a Wharton's Jelly-derived mesenchymal stem cell (abbreviated as WJMSC).

In some embodiments, the pre-conditioned mesenchymal stem cell has an expression level of a proliferative factor higher than that of a control mesenchymal stem cell unexposed to ginkgolide A. In some embodiments, the proliferative factor is phosphorylated protein kinase B (p-AKT), proliferating cell nuclear antigen (PCNA), cyclin E, cyclin B1, or any combination thereof. In some embodiments, the pre-conditioned mesenchymal stem cell has an expression level of a stemness factor higher than that of a control mesenchymal stem cell unexposed to ginkgolide A. In some embodiments, the stemness factor is octamer-binding transcription factor 4 (OCT4), Nanog, or a combination thereof. In some embodiments, the pre-conditioned mesenchymal stem cell has an expression level of a homing factor higher than that of a control mesenchymal stem cell unexposed to ginkgolide A. In some embodiments, the homing factor is C-X-C chemokine receptor 4 (CXCR4).

Also disclosed herein is a cell-protective composition for promoting recovery or reducing death of damaged nerve cells. The cell-protective composition includes the pre-conditioned mesenchymal stem cell mentioned above, the exosome derived therefrom, or a combination thereof. Optionally, the composition may further include a pharmaceutically acceptable carrier, a supplemental component, a second therapeutic agent, or any combination thereof.

In another aspect, the present disclosure provides a method for preparing a pre-conditioned mesenchymal stem cell or an exosome derived therefrom, including contacting a mesenchymal stem cell with an effective amount of ginkgolide A to obtain the pre-conditioned mesenchymal stem cell. Optionally, the method further includes isolating the exosome from a culture medium where the pre-conditioned mesenchymal stem cell is cultured.

In some embodiments, the mesenchymal stem cell to be treated with ginkgolide A is derived from bone marrow, blood, adipose tissues, muscle, skin, dental pulp, umbilical cord tissues, placenta, or amniotic fluid in a subject. In some embodiments, the mesenchymal stem cell is derived from Wharton's Jelly. In some embodiments, the contacting step is performed with ginkgolide A at a concentration of about 40 µM or more. In some embodiments, the contacting step lasts for about 24 hours or more.

In still another aspect, the present disclosure provides a method for promoting recovery or reducing death of damaged nerve cells, including administering to the damaged nerve cells a composition including the pre-conditioned mesenchymal stem cell or the exosome derived therefrom. The damaged nerve cells may have encountered oxidative stress.

In some embodiments, the damaged nerve cells suffer from mitochondrial dysfunction, autophagy dysregulation, apoptosis, or any combination thereof. In some embodiments, the exosome mitigates mitochondrial dysfunction, autophagy dysregulation, apoptosis, or any combination thereof, of the damaged nerve cells. In some embodiments, the exosome promotes alpha-synuclein clearance by the damaged nerve cells.

The pre-conditioned MSC disclosed herein exhibits enhanced proliferation, sternness, homing competence, and exosome-mediated functions in comparison to MSCs without pre-treatment with ginkgolide A. In addition, the pre-conditioned MSC and the exosome derived therefrom have been demonstrated to be more effective in protecting damaged nerve cells from death and repairing cellular dysfunctions of damaged nerve cells. Therefore, the pre-conditioned MSC and the thus produced exosome may be applied in treating neurodegenerative diseases, such as Parkinson's disease.

### BRIEF DESCRIPTION OF DRAWINGS

The present invention will be apparent to those skilled in the art from the following detailed description of the preferred embodiments, with reference to the attached drawings, in which:
FIG. 1A shows the cell viability of Wharton's Jelly-derived mesenchymal stem cells (WJMSCs) after exposure to 0, 20, 40, or 80 µM ginkgolide A (GA) for 1, 3, 5, or 7 days; the data are presented as mean ± standard deviation; ^{∗} indicates P < 0.05 compared to no GA treatment;
FIG. 1B shows the cell cycle distribution of WJMSCs untreated or treated with 80 µM GA; data are presented as mean ± standard deviation; ^{∗} indicates P < 0.05 compared to the untreated WJMSCs;
FIG. 1C shows the expression of proliferation-related proteins in WJMSCs after exposure to 0, 20, or 80 µM GA;
FIG. 1D shows the expression of cell cycle-related proteins in WJMSCs after exposure to 0, 20, or 80 µM GA;
FIG. 2A shows the homing capacity of WJMSCs after the indicated treatments; data are presented as the mean ± standard deviation; ^{∗} indicates P<0.05 between the marked two groups;
FIG. 2B shows the expression of homing proteins in WJMSCs after exposure to 0, 20, or 80 µM GA;
FIG. 3A shows the viability of SH-SY5Y cells after the indicated treatments; data are presented as mean ± standard deviation; ^{∗} indicates P < 0.05 compared to the 6-OHDA group;
FIG. 3B shows the viability of SH-SY5Y cells after the indicated treatments; data are presented as mean ± standard deviation; ^{∗} indicates P < 0.05 between the marked two groups;
FIG. 4A shows the size distribution of exosomes isolated from GA pre-treated WJMSCs or control WJMSCs that were unexposed to GA; the dark line shows the average size;
FIG. 4B shows the expression of positive and negative markers of exosomes isolated from GA pre-treated WJMSCs or the WJMSCs that were unexposed to GA; the abbreviation Exo stands for exosomes;
FIG. 4C shows the viability of SH-SY5Y cells after the indicated treatments; the abbreviation Exo stands for exosomes; data are presented as mean ± standard deviation; ^{∗} indicates P < 0.05 between the marked two groups;
FIG. 4D shows the dot plots of SH-SY5Y cells treated as indicated and analyzed by flow cytometry; the SH-SY5Y cells were stained with annexin V-FITC and propidium iodide (PI); the abbreviation Exo stands for exosomes;
FIG. 4E shows the proportion of apoptotic SH-SY5Y cells calculated based on the data of Fig. 4D; the abbreviation Exo stands for exosomes; data are presented as mean ± standard deviation; ^{∗} indicates P < 0.05 between the marked two groups;
FIG. 4F shows the number of TUNEL positive SH-SY5Y cells after the indicated treatments; the abbreviation Exo stands for exosomes; data are presented as mean ± standard deviation; ^{∗} indicates P < 0.05 compared to the control group; # indicates P < 0.05 compared to the 6-OHDA group;
FIG. 5A shows the expression of apoptosis-related proteins and anti-apoptotic proteins in SH-SY5Y cells after the indicated treatments; the abbreviation Exo stands for exosomes;
FIG. 5B shows the expression of apoptosis-related proteins in SH-SY5Y cells after the indicated treatments; the abbreviation Exo stands for exosomes;
FIG. 5C shows the expression of mitochondrial oxidative phosphorylation proteins in SH-SY5Y cells after the indicated treatments; the abbreviation Exo stands for exosomes;
FIG. 6A shows the images of SH-SY5Y cells captured by a fluorescence microscope after the indicated treatments and immunofluorescence staining; the abbreviation Exo stands for exosomes; the white arrows indicate increased LC3 puncta;
FIG. 6B shows the expression of autophagy-related proteins in SH-SY5Y cells after the indicated treatments; the abbreviation Exo stands for exosomes; and
FIG. 6C shows the images of SH-SY5Y cells captured by a fluorescence microscope after the indicated treatments and immunofluorescence staining; the abbreviation Exo stands for exosomes; the white arrows indicate the presence of alpha-synuclein phosphorylated at serine 129 (P-S129 alpha-synuclein).

### DETAILED DESCRIPTION OF THE INVENTION

The present invention is further explained in the following embodiments and examples. It is understood that the examples given below do not limit the scope of the invention, and it will be evident to those skilled in the art that modifications can be made without departing from the scope of the appended claims.

Unless defined otherwise, all technical and scientific terms and abbreviations used herein have the same meaning as commonly understood by a person skilled in the art to which this invention pertains.

### Definition

As used herein, the singular forms "a", "an" and "the" include plural referents unless the context clearly indicates otherwise.

Numerical quantities given herein are approximate, and experimental values may vary within 20 percent, preferably within 10 percent, and most preferably within 5 percent. Thus, the terms "about" and "approximately" refer to within 20 percent, preferably within 10 percent, and most preferably within 5 percent of a given value or range.

The terms "mesenchymal stem cell(s)" and "MSC(s)" as used herein are interchangeable and refer to multipotent fetal or adult stem cells that can self-renew by division and can differentiate into multiple tissues, including bone, cartilage, adipose tissue, muscle, and non-mesodermal cells (for example, neural cells). The MSCs may be directly isolated from various tissues such as bone marrow in a subject. The MSCs may also be obtained through expansion of isolated MSCs or derivation from other stem cells such as embryonic stem cells (ESC) and induced pluripotent stem cells (iPSC). The MSCs are usually characterized by the expression of a first group of surface markers including CD90, CD73, and CD105 and the absence of a second group of surface markers including CD34, CD14, and CD45.

The term "exosome(s)" as used herein refers to a class of membrane-derived extracellular vesicles (about 30-150 nm in diameter) released by various cells. The exosomes act as mediators of intercellular communication by delivering bioactive molecules between cells through direct or indirect contact. The bioactive molecules may include DNAs, mRNAs, non-coding RNAs, proteins, and lipids.

As used herein, the term "subject" refers to a mammal. The subject may be human or non-human, including but not limited to a primate, murine, dog, cat, bovine, goat, horse, rabbit, pig, or the like.

As used herein, the term "nerve cells" refers to the cells constituting the nervous system, including but not limited to neurons and glial cells. The nerve cells may be mature nerve cells or immature nerve cells that can develop into mature nerve cells, such as neuroblasts.

The expression "effective amount of ginkgolide A" as used herein refers to the amount of ginkgolide A required to enhance the proliferation, sternness, homing competence, and/or exosome-mediated functions of MSCs. As appreciated by those skilled in the art, the effective amount will vary depending on the source of MSCs and the culturing conditions of MSCs.

The terms "culture" and "culturing" as used herein refer to incubating cells under in vitro conditions that allow for cell growth or division or to maintain cells in a living state.

### Pre-conditioned MSCs, exosomes, and preparation methods thereof

The present disclosure provides a pre-conditioned mesenchymal stem cell or an exosome derived therefrom, wherein the pre-conditioned mesenchymal stem cell is an isolated mesenchymal stem cell pre-exposed to ginkgolide A.

The present disclosure also provides a method for preparing a pre-conditioned mesenchymal stem cell or an exosome derived therefrom, including contacting an isolated mesenchymal stem cell with an effective amount of ginkgolide A to obtain the pre-conditioned mesenchymal stem cell.

Prior to exposure to ginkgolide A, the MSC can be obtained from diverse sources, including bone marrow, blood, adipose tissues, muscle, skin, dental pulp, umbilical cord tissues, placenta, or amniotic fluid in a subject such as a human subject. In some embodiments, the MSC is derived from Wharton's Jelly. The MSC may be freshly isolated from the source organs or tissues and then cultured by methods known to those skilled in the art. The MSC may also be commercially available.

The pre-conditioned MSC may be obtained by contacting an MSC with ginkgolide A at a predetermined concentration for a predetermined period of time. Ginkgolide A may be purchased from chemical suppliers such as Merck (Germany). Before use, ginkgolide A may be dissolved in an organic solvent such as dimethyl sulfoxide (DMSO) or alcohols to form a solution and then be diluted with an aqueous solution such as a saline or a phosphate buffer. In some embodiments, ginkgolide A is added to a culture medium where the MSC is cultured, wherein the ginkgolide A is at a concentration approximately between 40 µM and 50 µM, between 50 µM and 60 µM, between 60 µM and 70 µM, between 70 µM and 80 µM, or above 80 µM. In some embodiments, the predetermined period of time for contacting with ginkgolide A is approximately between 24 hours and 1.5 days, between 1.5 and 2 days, between 2 and 2.5 days, between 2.5 and 3 days, between 3 and 3.5 days, between 3.5 and 4 days, between 4 and 4.5 days, between 4.5 and 5 days, between 5 and 5.5 days, between 5.5 and 6 days, between 6 and 6.5 days, between 6.5 and 7 days, or above 7 days.

In some embodiments, the MSC is in contact with ginkgolide A during the expansion of the MSC. In other embodiments, the MSC is exposed to ginkgolide A after the cell expansion.

The pre-conditioned MSC exhibits enhanced proliferation, sternness, homing competence, and/or exosome-mediated functions when compared to a control mesenchymal stem cell unexposed to ginkgolide A. The enhanced proliferation, sternness, and homing competence may be examined by methods well-known to those skilled in the art, for example, biochemical assays that can measure the expression level of a protein in cells and other techniques that can determine cell expansion or cell cycle phases or analyze cell morphology or migration.

In some embodiments, the pre-conditioned mesenchymal stem cell has an expression level of a proliferative factor higher than that of a control mesenchymal stem cell unexposed to ginkgolide A. The term "proliferative factor" refers to a protein produced by cells marking cell proliferation or division. Examples of the proliferative factor include but are not limited to kinases (such as p-AKT), proteins for promoting DNA replication (such as PCNA), and proteins for controlling cell cycle progression (such as cyclins).

In some embodiments, the pre-conditioned mesenchymal stem cell has an expression level of a sternness factor higher than that of a control mesenchymal stem cell unexposed to ginkgolide A. The term "sternness factor" refers to a protein produced by stem cells that is crucial for maintaining pluripotency or multipotency. Examples of the sternness factor include but are not limited to proteins involving in the self-renewal signaling pathway of stem cells, such as OCT4 and Nanog.

In some embodiments, the pre-conditioned mesenchymal stem cell has an expression level of a homing factor higher than that of a control mesenchymal stem cell unexposed to ginkgolide A. The term "homing factor" refers to a protein produced by cells marking the ability of stem cells to migrate between two sites, for example, migrating from the site where stem cells are transplanted to injured tissues. Examples of the homing factor include but are not limited to chemokine receptors (such as CXCR4) and adhesion molecules (such as integrins).

Once the pre-conditioned MSC is obtained, exosomes can be prepared from any fluids that have been used to incubate the pre-conditioned MSC. In some embodiments, the exosome is isolated from a culture medium where the pre-conditioned MSC is cultured. Various methods known in the art can be utilized to isolate exosomes, for example, centrifugation (such as differential centrifugation and density gradient centrifugation), chromatography (such as size-exclusion chromatography), filtration with filtration membranes, polymer-based precipitation, and immunological separation using biomolecules recognizing exosomal surface proteins.

The isolated exosomes may be characterized by various approaches well-known in the art, including biophysical approaches and molecular approaches. Biophysical methods characterize the size of exosomes. One example of the biophysical approach is optical particle tracking, which measures the size distribution of exosomes from 10 nm to 2 µm and the concentration of exosomes. Molecular approaches are used for characterizing exosomal surface proteins. For example, flow cytometry can measure the size and chemical structure of exosomes that are labeled with dyes or fluorophore-conjugated antibodies. Western blot can be utilized to detect the presence of exosome positive markers.

### Cell-protective compositions

The present disclosure also provides a cell-protective composition for promoting recovery or reducing death of damaged nerve cells. In some embodiments, the cell-protective composition includes the pre-conditioned MSC described herein. The pre-conditioned MSC may be a specific pre-conditioned MSC or a composition of pre-conditioned MSCs, for example, a composition including a first and a second pre-conditioned MSCs of different origins. In the case where the cell-protective composition contains a composition of pre-conditioned MSCs, even if the pre-conditioned MSCs have different molecular constituents individually (such as different protein expression profiles), they collectively show enhanced proliferation, sternness, homing competence, and/or exosome-mediated functions when compared to a control mesenchymal stem cell unexposed to ginkgolide A.

Alternatively, the cell-protective composition may include an exosome derived from the pre-conditioned MSC. The exosome may be a specific exosome or a composition of exosomes. In the case where the cell-protective composition contains a composition of exosomes, even if the exosomes have different molecular constituents individually, they collectively can promote the recovery of damaged nerve cells or protect the damaged cells from death more effectively when compared to the exosomes generated by MSCs unexposed to ginkgolide A.

In some embodiments, the cell-protective composition includes a pre-conditioned MSC and an exosome derived therefrom in combination. In some embodiments, the cell-protective composition includes a first pre-conditioned MSC and an exosome derived from a second pre-conditioned MSC.

Optionally, the cell-protective composition may further include a pharmaceutically acceptable carrier, a supplemental component, a second therapeutic agent, or any combination thereof. The selection and amount for use of these substances may vary depending on the application of the cell-protective composition.

In some embodiments, the cell-protective composition further includes a pharmaceutically acceptable carrier that is widely used in the field of pharmaceutical manufacturing. The pharmaceutically acceptable carrier may be one or more agents selected from solvents (such as water, saline, aqueous buffers, alcohols, and organic solvents), emulsifiers, suspending agents, stabilizing agents, diluents, and preservatives.

In some embodiments, the cell-protective composition further includes a supplemental component such as a culture medium or an MSC-derived product. The culture medium refers to a solid, semi-solid, or liquid composition including water and nutrients (for example, amino acids, glucose, and salts) to support the living of cells and optionally including a matrix component such as agarose, gelatin, and collagen. The culture medium may have or have not been used for culturing the pre-conditioned MSC. The MSC-derived product refers to any molecules or molecular complexes generated and released by MSCs exposed or unexposed to ginkgolide A. Examples of the MSC-derived product include but are not limited to proteins, nucleic acids, lipids, and extracellular vesicles.

In some embodiments, the cell-protective composition further includes a second therapeutic agent. The term "therapeutic agent" refers to a pharmaceutically active ingredient that results in a favorable response in a recipient after administration. The second therapeutic agent may be a small-molecular drug or a macromolecule such as proteins or nucleic acids. Examples of the therapeutic agent include but are not limited to growth factors, antioxidants, and immunomodulatory agents.

The composition described herein may be administered via various routes, including but not limited to direct contact, intravenous injection, or site injection. Depending on the route of administration, the cell-protective composition may be formulated, by techniques well-known to those skilled in the art, into a suitable dosage form, including but not limited to injections, suspensions, emulsions, and the like.

### Uses of the pre-conditioned MSCs or the exosomes

The present disclosure also provides a method for promoting recovery or reducing death of damaged nerve cells, including administering to the damaged nerve cells a therapeutically effective amount of a composition including the pre-conditioned MSC disclosed herein or an exosome derived therefrom. The term "recovery" refers to returning to a normal state of health at the cell level, which can be determined by examination of morphological features of cells (such as cell membrane integrity) or assessment of various cellular functions, for example, protein and DNA synthesis, nutrient metabolism, energy production, and elimination of unnecessary cell components. The expression "therapeutically effective amount" refers to the amount of the composition including the pre-conditioned MSC or the pre-conditioned MSC-derived exosomes that is sufficient to alleviate the morphological or functional abnormality of damaged nerve cells or to reduce cell death.

The damaged nerve cells refer to nerve cells that are injured due to extracellular or intracellular environmental stimuli in vivo or in vitro. The environmental stimuli may be physical, chemical, biological, nutritional, microbial, or immunological stimuli. Generally, the structures and functions of cellular components (such as genetic materials, proteins, cell membrane, and mitochondria) in the damaged nerve cells would alter and thus deviate from the normal state of health. Sometimes cell death (for example, through apoptosis or autophagy) occurs if the injury is too severe for the cells to recover.

In some embodiments, the damaged nerve cells have encountered oxidative stress. The term "oxidative stress" refers to a state of excess accumulation of oxidizing species (such as free radicals) due to increased production of reactive oxygen species (ROS) and/or reduced clearance of the oxidizing species by cells. Oxidative stress may be triggered by various factors, including exposure to chemicals that would induce ROS production, exposure to radiation, inflammation, and a decrease in the cellular production of antioxidants.

In some embodiments, the damaged nerve cells suffer from mitochondrial dysfunction, autophagy dysregulation, apoptosis, or any combination thereof. In some embodiments, the pre-conditioned MSC-derived exosome mitigates mitochondrial dysfunction, autophagy dysregulation, apoptosis, or any combination thereof, of the damaged nerve cells. In some embodiments, the pre-conditioned MSC-derived exosome promotes alpha-synuclein clearance by the damaged nerve cells. Since the aggregation of alpha-synuclein has been reported to play a role in the development of Parkinson's disease, the ability of the exosome to reduce alpha-synuclein aggregation demonstrates their neuroprotective effects on nerve cells.

The following examples are provided for those skilled in the art to understand the present invention more easily but are not intended to limit the present invention.

### Example 1

### Ginkgolide A (GA) promotes proliferation and stemness of MSCs

In this and the following examples, human Wharton's Jelly-derived MSCs (WJMSCs) were obtained from Cellular Engineering Technologies Inc (Coralville, Iowa, USA). The WJMSCs were incubated in a humidified incubator at 37°C with 5% CO₂. Dulbecco's modified essential medium (DMEM; Life Technologies, USA) supplemented with 10% fetal bovine serum (FBS; Hyclone, Thermo Fisher Scientific, USA) and 1% penicillin-streptomycin (Gibco, Thermo Fisher Scientific, USA) was used for culturing of the WJMSCs.

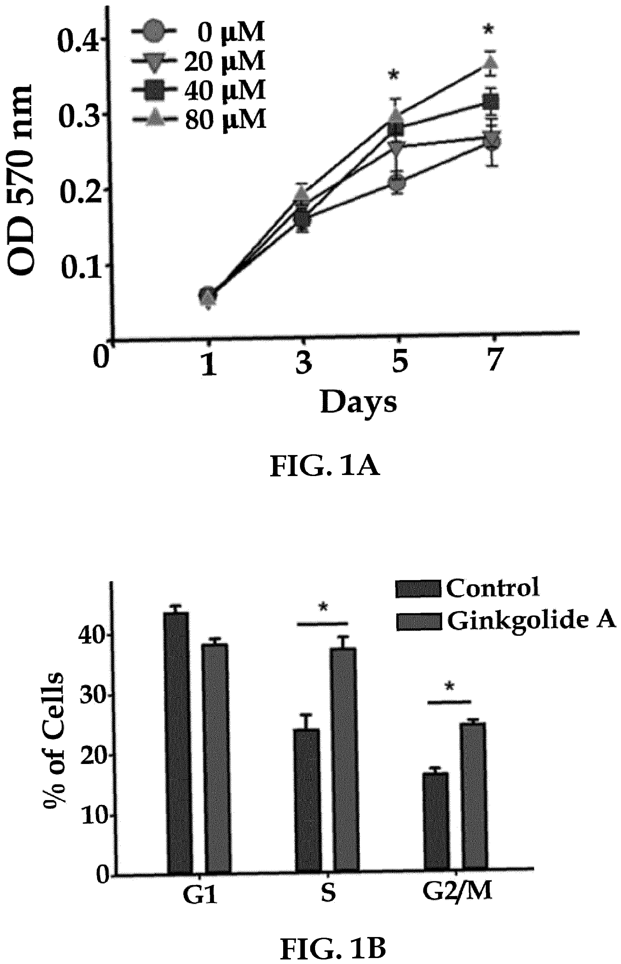

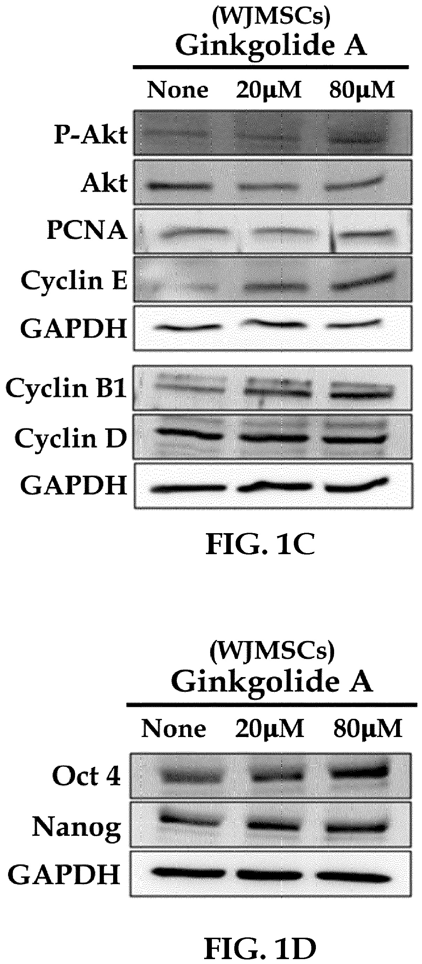

The effects of GA on MSC proliferation were investigated by treating the WJMSCs with different concentrations of GA (0, 20, 40, or 80 µM) for 1, 3, 5, or 7 days and estimating the number of viable cells by MTT (3-(4,5-dimethylthiazol-2-yl)-2,5-diphenyltetrazolium bromide) assay. Before cell viability was determined spectrophotometrically at a wavelength of 570 nm (expressed as OD, i.e., optical density), the WJMSCs treated differently were washed with phosphate-buffered saline (PBS) and incubated with an MTT reagent for 2 hours. As shown in FIG. 1A, treatment with 40 µM or more GA for five days significantly enhanced the proliferation of the WJMSCs in comparison to control WJMSCs that were unexposed to GA. The changes in cell cycle phases of the WJMSCs after treatment with 80 µM GA were further assessed by flow cytometry. As shown in FIG. 1B, exposure to GA changed the cell cycle distribution in S and G2/M phases as compared to that in control WJMSCs.

In addition, the expression of proliferation and cell cycle-related proteins in the WJMSCs was evaluated by western blotting. As shown in FIG. 1C, the expression level of p-AKT, PCNA, Cyclin E, and Cyclin B1 increased after GA treatment (80 µM GA for 24 hours). GA also enhanced the expression of sternness-related proteins such as OCT4 and Nanog in WJMSCs (FIG. ID). These findings indicated that GA could improve the proliferation and stemness of MSCs, thus allowing the preparation of the pre-conditioned MSC disclosed herein.

### Example 2

### GA enhances the homing capability of MSCs

In this and the following examples, human neuroblastoma cell line SH-SY5Y (a cell model of dopaminergic neurons for Parkinson's disease research) that was damaged by 6-hydroxydopamine (6-OHDA, i.e., 2,4,5-trihydroxyphenethylamine)-induced oxidative stress was used to assess the effects of GA on the homing ability of MSCs and the neuroprotective effects of GA pre-treated MSCs and the exosomes derived therefrom. SH-SY5Y cells were obtained from the American Type Culture Collection (ATCC; Manassas, Virginia, USA). The SH-SY5Y cells were cultured in a humidified incubator at 37°C with 5% CO₂. DMEM (Life Technologies, USA) supplemented with 10% FBS (Hyclone, Thermo Fisher Scientific, USA) and 1% penicillin-streptomycin (Gibco, Thermo Fisher Scientific, USA) was used for culturing of the SH-SY5Y cells.

To examine in vitro homing ability, WJMSCs (2 × 10⁵ cells) pre-treated with or without 80 µM GA for 24 hours were seeded in the upper chamber of a transwell insert, and SH-SY5Y cells (3 × 10⁴ cells) were seeded in the bottom well of the transwell plate where 500 µL culture medium containing 6-OHDA (100 µM) were placed in the bottom well. The cells were co-cultured at 37°C to allow WJMSC migration through a porous membrane (0.4 µm) of the transwell insert. After 24-hour incubation, the non-migratory WJMSCs were removed by a cotton swab from one side of the transwell membrane. The migratory WJMSCs adhered to the other side of the transwell membrane were fixed with 4% paraformaldehyde (PFA), stained with crystal violet, and quantified. The migration ability of WJMSCs was expressed as fold-change compared to a control group (i.e., WJMSCs untreated with GA and SH-SY5Y cells without 6-OHDA stimulation were co-cultured).

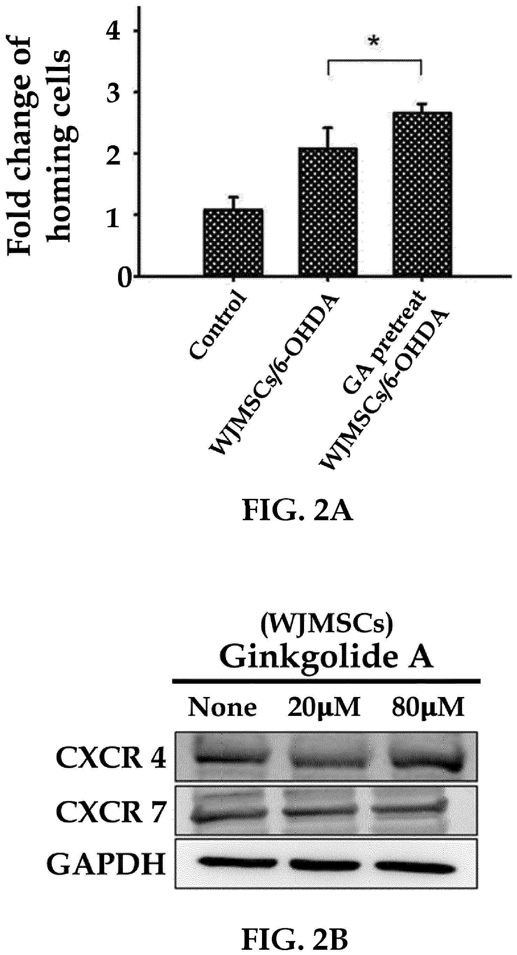

As shown in FIG. 2A, the GA pre-treated WJMSCs displayed higher migrating capacity compared to the untreated WJMSCs. Moreover, as shown in FIG. 2B, GA enhanced the expression of the homing protein CXCR4 in WJMSCs. These results suggested that GA could promote WJMSCs homing ability through the SDF-1/CXCR4 signaling pathway.

### Example 3

### GA pre-treated MSCs protect damaged nerve cells from cell death

To determine whether GA can promote MSCs to rescue nerve cell death, transwell co-culture assay was performed where WJMSCs (2 × 10⁴ cells) pre-treated with 0, 20, 40, or 80 µM GA for 24 hours were placed in the upper chamber of a transwell insert, and SH-SY5Y cells (3 × 10⁴ cells) treated with 100 µM 6-OHDA were seeded in the bottom well of the transwell plate. After co-culture of the cells at 37°C for 24 hours, the SH-SY5Y cell viability was determined by MTT assay.

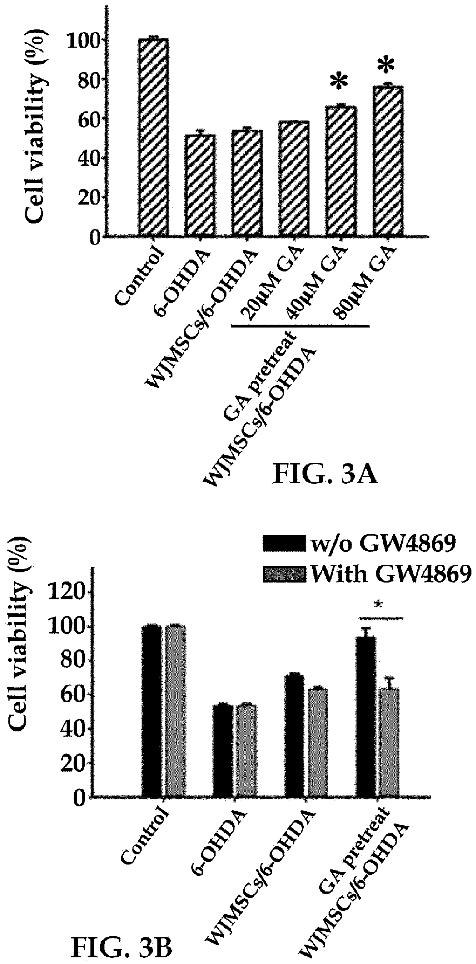

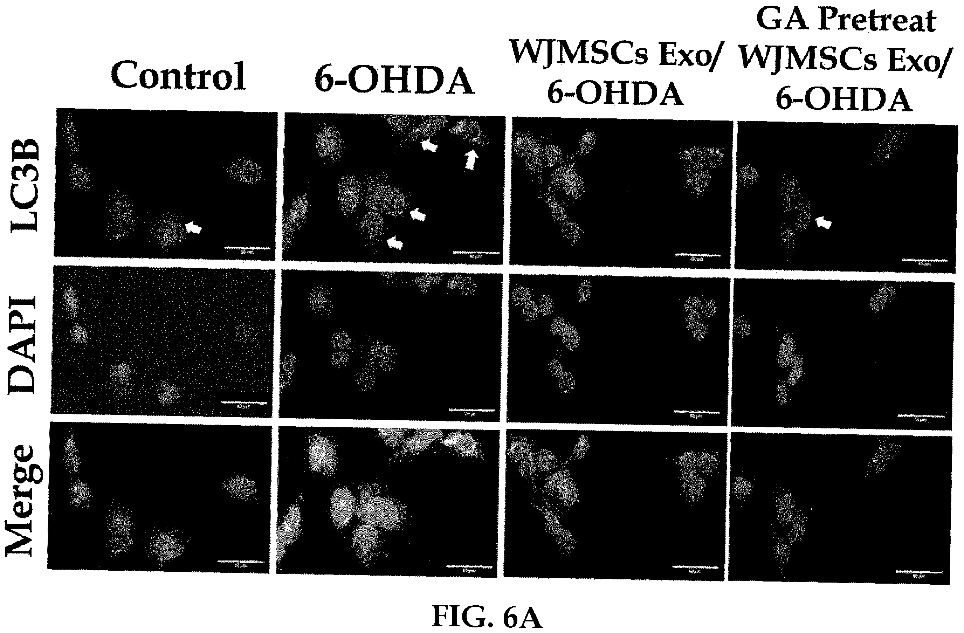

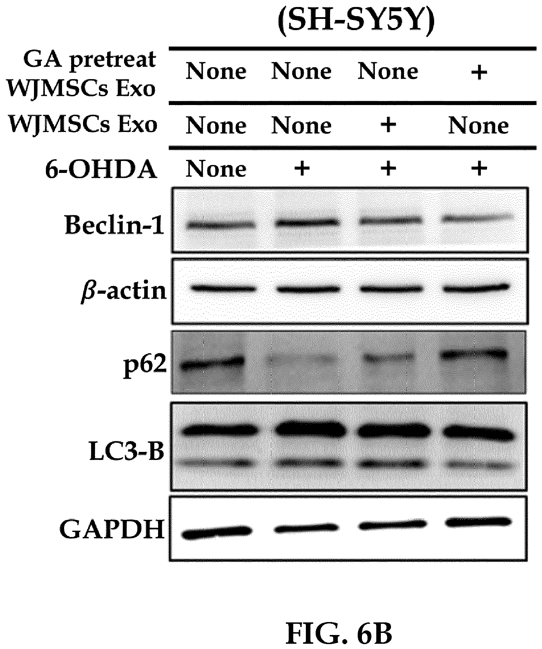

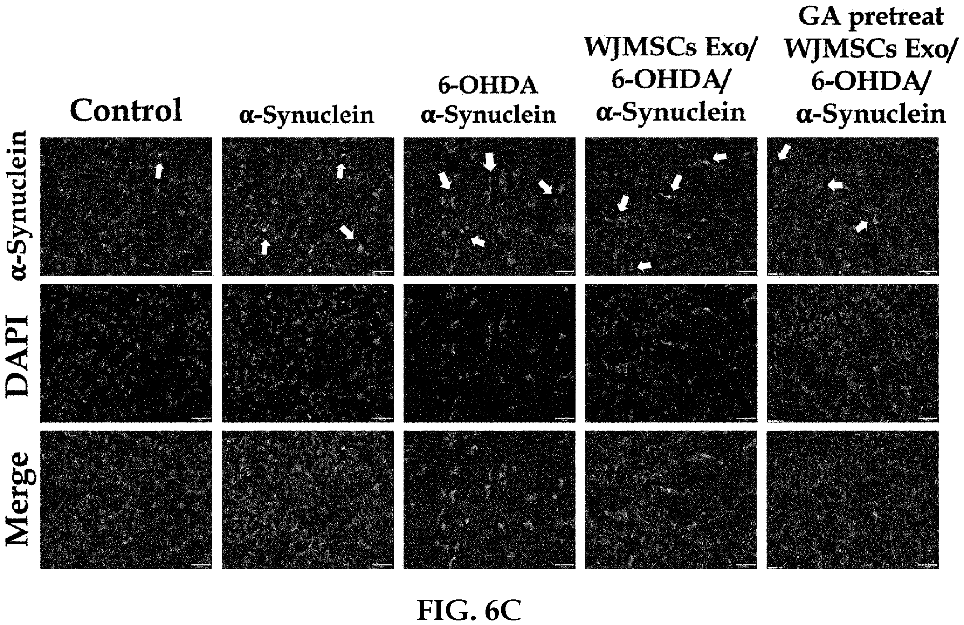

As shown in FIG. 3A, 6-OHDA decreased SH-SY5Y cell viability, whereas GA pre-treated WJMSCs significantly inhibited the death of SH-SY5Y cells under 6-OHDA stimulation. These findings suggested that GA pre-treated WJMSCs repaired the damage probably through secretomes, cytokines, or exosomes. To further clarify the mode of action of the GA pre-treated WJMSCs, GW4869, an inhibitor of exosome generation, was utilized in the co-culture assay to block exosome production by WJMSCs. As shown in FIG. 3B, the protective effect of the GA pre-treated WJMSCs was suppressed in the presence of GW4869, suggesting that exosomes contribute to the neuroprotective effects of GA pre-treated MSCs.

### Example 4

### GA pre-treated MSC-derived exosomes inhibit apoptosis of damaged nerve cells

To study the exosome-mediated functions of MSCs enhanced by GA, exosomes were isolated from WJMSCs pre-treated with or without 80 µM GA for 24 hours. In brief, exosomes were collected from the culture medium where WJMSCs were cultured using an Exosome Purification Kit (Exo-spin; Cell Guidance Systems, USA) according to the manufacturer's instructions. The culture medium was centrifuged at 300 × g for 10 minutes to remove cells and debris. The supernatant was then transferred to a fresh centrifuge tube and spun at 20,000 × g for 30 minutes to remove any remaining cell debris. The exosomes were then precipitated at 4°C for 24 hours. The exosome-containing pellet was resuspended in 200 µL PBS and added to the Exo-spin column. The exosomes were then eluted from the column with PBS, and the collection tube containing the isolated exosomes was centrifuged at 50 × g for 60 seconds to collect all liquid to the bottom of the tube.

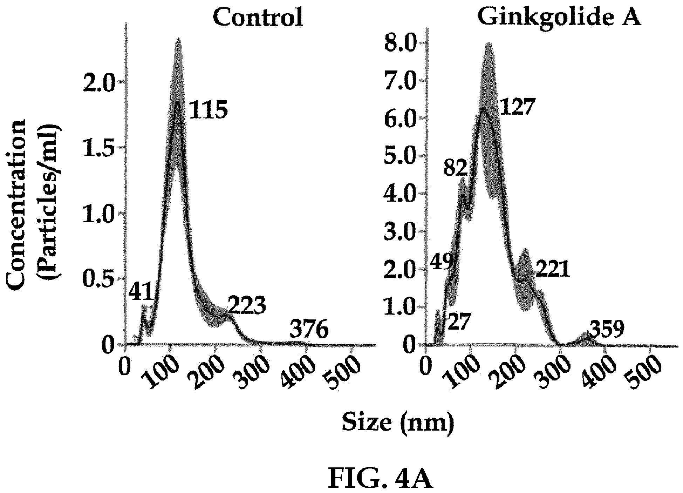

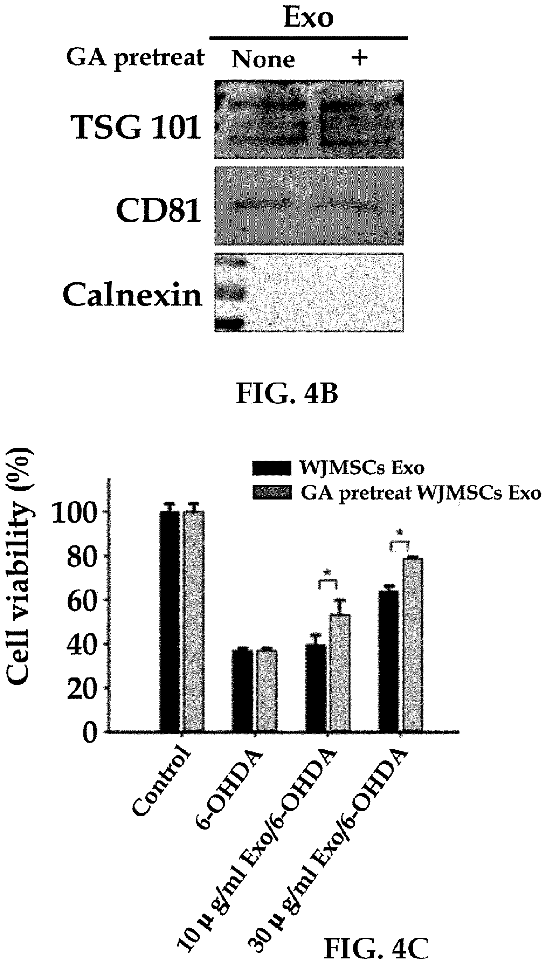

FIG. 4A shows the estimated diameter of the isolated exosomes measured by a nanoparticle size analyzer (NanoSight NS300; Malvern Panalytical). The GA pre-treated WJMSC-derived exosomes had an average size larger than that of the exosomes isolated from the control WJMSCs unexposed to GA. Further, the isolated exosomes were verified by the presence of exosome positive markers such as tumor susceptibility gene 101 (TSG101) and CD81 and the absence of exosome negative markers such as calnexin using western blotting (FIG. 4B).

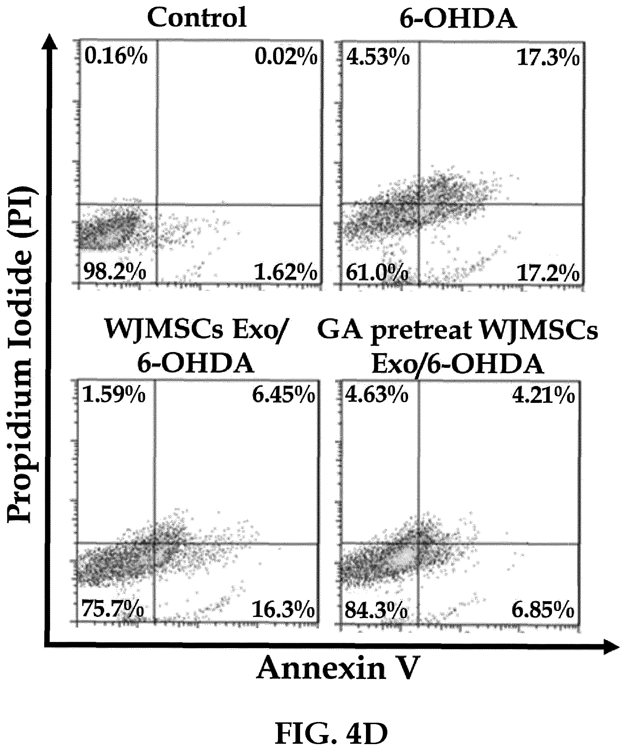

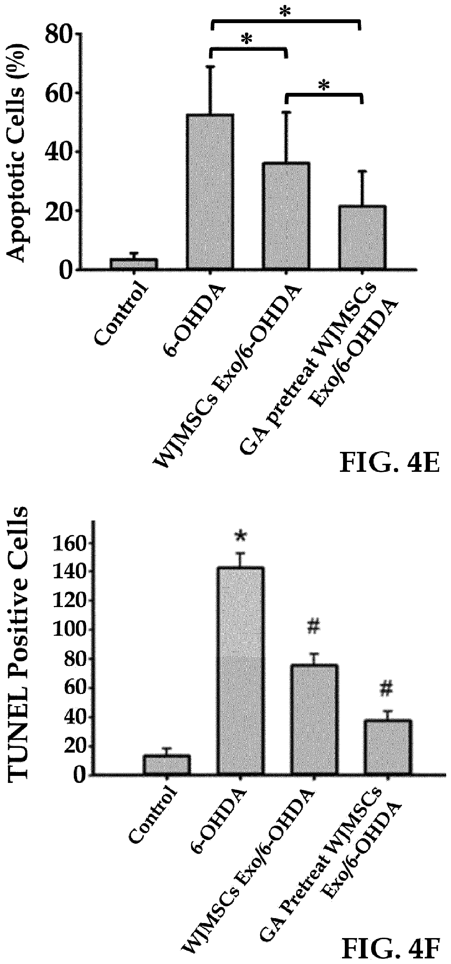

FIG. 4C shows the SH-SY5Y cell viability measured by MTT assay. It was found that the exosomes derived from the GA pre-treated WJMSCs protected more SH-SY5Y cells from death under 6-OHDA stimulation compared to the exosomes derived from the WJMSCs that were unexposed to GA. In addition, flow cytometry and TUNEL assay were employed to measure apoptotic cell death. FIG. 4D shows the dot plots of SH-SY5Y cells analyzed by a flow cytometry (BD FACSLyric system; BD Biosciences). FIG. 4E shows the proportion of apoptotic SH-SY5Y cells calculated based on the data of FIG. 4D. FIG. 4F shows the number of TUNEL positive SH-SY5Y cells. All these data demonstrated that the GA pre-treated WJMSC-derived exosomes inhibited SH-SY5Y cell apoptosis more effectively compared to the exosomes derived from the untreated WJMSCs (FIGs. 4D, 4E, and 4F).

### Example 5

### GA pre-treated MSC-derived exosomes suppress the signaling for apoptosis and restore mitochondrial homeostasis in damaged nerve cells

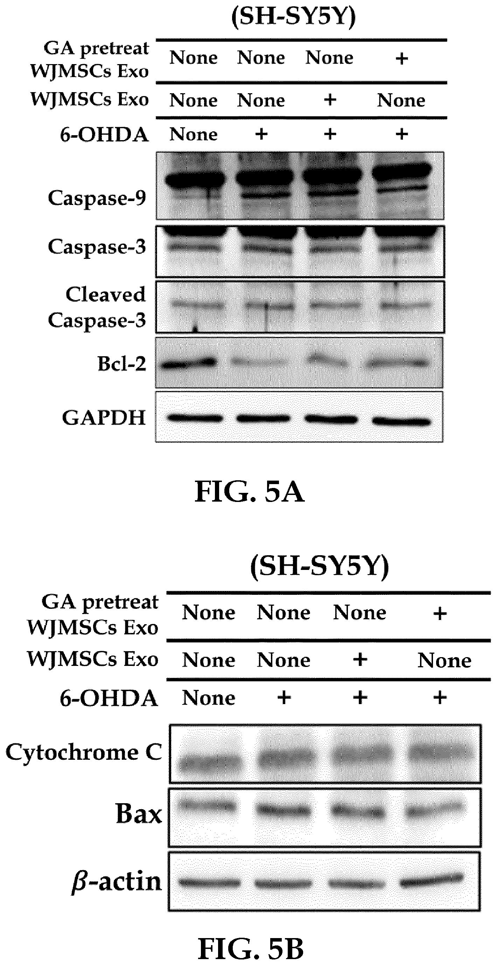

To further investigate the molecular events associated with the anti-apoptotic effects of GA pre-treated MSC-derived exosomes, the expression of apoptosis-related and anti-apoptotic proteins in 6-OHDA-stimulated SH-SY5Y cells was measured using western blotting. The damaged SH-SY5Y cells were treated with exosomes isolated either from GA pre-treated WJMSCs (80 µM GA for 24 hours) or untreated WJMSCs. As shown in FIG. 5A and FIG. 5B, the GA pre-treated WJMSC-derived exosomes reduced the expression of apoptosis-related proteins such as caspase-9, cleaved caspase-3, cytochrome C, and Bax in damaged SH-SY5Y cells while also increasing the expression of anti-apoptotic proteins such as Bcl-2.

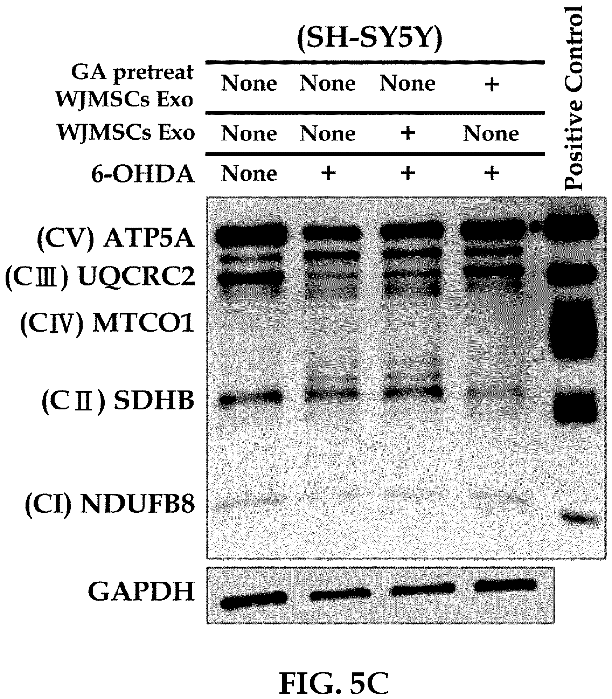

Moreover, the expression of mitochondrial proteins involved in oxidative phosphorylation (OXPHOS), including ATP5A, UQCRC2, MTCO1, SDHB, and NDUFB8, was examined by western blotting. FIG. 5C shows that 6-OHDA treatment downregulated the expression of the OXPHOS proteins, but the exosomes isolated from the GA pre-treated WJMSCs restored the mitochondrial homeostasis in the damaged SH-SY5Y cells.

### Example 6

### GA pre-treated MSC-derived exosomes restore autophagy in damaged nerve cells and promote alpha-synuclein clearance

Increasing evidence suggests that dysregulation of autophagy results in the accumulation of abnormal proteins, which is commonly observed in brain tissues of patients with neurodegenerative diseases. Hence, the effects of GA pre-treated MSC-derived exosomes on autophagic flux in damage nerve cells were assessed by immunofluorescence and western blotting. SH-SY5Y cells under 6-OHDA stimulation were treated with exosomes isolated either from GA pre-treated WJMSCs (80 µM GA for 24 hours) or untreated WJMSCs. For immunofluorescence staining, the treated cells were fixed in 4% PFA, incubated with an anti-LC3B primary antibody followed by a fluorophore-conjugated secondary antibody, and stained with 4',6-diamidino-2-phenylindole (DAPI). The SH-SY5Y cells were then visualized by a fluorescence microscope (Olympus).

As shown in FIG. 6A, LC3 puncta characterizing the formation of autophagosomes were abundant in SH-SY5Y cells under 6-OHDA stimulation. However, the LC3 puncta in damaged SH-SY5Y cells after treatment with GA pre-treated WJMSC-derived exosomes were reduced to a level close to that of control SH-SY5Y cells without 6-OHDA stimulation. In addition, as shown in FIG. 6B, increased Beclin-1 and LC3B expression but decreased p62 protein expression were observed under 6-OHDA stimulation, indicating autophagy dysregulation. However, treatment with the exosomes from GA pre-treated WJMSCs restored autophagy to an extent comparable to that for control SH-SY5Y cells.

To further validate the findings described above, SH-SY5Y cells were treated with active human recombinant alpha-synuclein pre-formed fibrils (5 µM; GeneTex) under 6-OHDA stimulation to induce alpha-synuclein aggregation. The presence of alpha-synuclein was detected by immunofluorescence staining with an antibody against P-S129 alpha-synuclein. As shown in FIG. 6C, alpha-synuclein aggregation was reduced more significantly after administering GA pre-treated WJMSCs-derived exosomes, compared to exosomes derived from untreated WJMSCs, suggesting that GA pre-treated WJMSCs-derived exosomes could promote alpha-synuclein clearance through improving autophagic flux in damaged nerve cells.

The pre-conditioned MSC prepared by pre-exposure to ginkgolide A displays enhanced proliferation, sternness, homing competence, and exosome-mediated functions and can protect damaged nerve cells from death more effectively than MSCs unexposed to ginkgolide A. Moreover, the exosome derived from the pre-conditioned MSC can modulate abnormal activities in damaged nerve cells to suppress progressive nerve cell death, which signifies neurodegenerative diseases. Therefore, the pre-conditioned MSC and the exosome derived therefrom may be utilized to manufacture a cell-protective composition such as a pharmaceutical composition for treating neurodegenerative diseases.

## Claims

1. A pre-conditioned mesenchymal stem cell or an exosome derived therefrom, wherein the pre-conditioned mesenchymal stem cell is a mesenchymal stem cell pre-exposed to ginkgolide A.

2. The pre-conditioned mesenchymal stem cell or the exosome according to claim 1, wherein the mesenchymal stem cell is derived from bone marrow, blood, adipose tissues, muscle, skin, dental pulp, umbilical cord tissues, placenta, or amniotic fluid in a subject.

3. The pre-conditioned mesenchymal stem cell or the exosome according to claim 1 or claim 2, wherein the pre-conditioned mesenchymal stem cell has an expression level of a proliferative factor higher than that of a control mesenchymal stem cell unexposed to the ginkgolide A, and/or wherein the pre-conditioned mesenchymal stem cell has an expression level of a stemness factor higher than that of a control mesenchymal stem cell unexposed to the ginkgolide A, and/or wherein the pre-conditioned mesenchymal stem cell has an expression level of a homing factor higher than that of a control mesenchymal stem cell unexposed to the ginkgolide A.

4. The pre-conditioned mesenchymal stem cell or the exosome according to claim 3, wherein the proliferative factor is p-AKT, PCNA, Cyclin E, Cyclin B1, or any combination thereof.

5. The pre-conditioned mesenchymal stem cell or the exosome according to claim 3 or claim 4, wherein the stemness factor is OCT4, Nanog, or a combination thereof.

6. The pre-conditioned mesenchymal stem cell or the exosome according to any one of claims 3 to 5, wherein the homing factor is CXCR4.

7. A cell-protective composition, comprising the pre-conditioned mesenchymal stem cell or the exosome according to any one of claims 1-6, or a combination thereof, or further comprising a pharmaceutically acceptable carrier, a supplemental component, a second therapeutic agent, or any combination thereof.

8. A method for preparing a pre-conditioned mesenchymal stem cell or an exosome derived therefrom, comprising contacting a mesenchymal stem cell with an effective amount of ginkgolide A to obtain the pre-conditioned mesenchymal stem cell.

9. The method of claim 8, wherein the mesenchymal stem cell is derived from bone marrow, blood, adipose tissues, muscle, skin, dental pulp, umbilical cord tissues, placenta, or amniotic fluid in a subject.

10. The method of claim 8 or claim 9, wherein the ginkgolide A is at a concentration of about 40 µM or more.

11. The method of any one of claims 8 to 10, wherein the contacting lasts for about 24 hours or more.

12. The method of any one of claims 8 to 11, further comprising isolating the exosomes from a culture medium where the pre-conditioned mesenchymal stem cell is cultured.

13. A method for promoting recovery or reducing death of damaged nerve cells, comprising administering to the damaged nerve cells a composition comprising the pre-conditioned mesenchymal stem cell or the exosome according to claim 1 or claim 2.

14. The method of claim 13, wherein the exosome mitigates mitochondrial dysfunction, autophagy dysregulation, apoptosis, or any combination thereof, of the damaged nerve cells.

15. The method of claim 13 or claim 14, wherein the exosome promotes alpha-synuclein clearance by the damaged nerve cells.
